(19) Europäisches Patentamt
European Patent Office
Office européen des brevets

(11) Publication number : 0 194 146 B2

(12) NEW EUROPEAN PATENT SPECIFICATION

(45) Date of publication of the new patent specification : 26.01.94 Bulletin 94/04

(51) Int. Cl.$^5$ : C07D 275/02, A01N 43/80

(21) Application number : 86301579.8

(22) Date of filing : 06.03.86

# (54) Stabilization of 5-chloro-3-isothiazolones.

(30) Priority : 08.03.85 US 709755

(43) Date of publication of application : 10.09.86 Bulletin 86/37

(45) Publication of the grant of the patent : 24.05.89 Bulletin 89/21

(45) Mention of the opposition decision : 26.01.94 Bulletin 94/04

(84) Designated Contracting States : AT BE CH DE FR GB IT LI LU NL SE

(56) References cited :
EP-A- 0 166 611
GB-A- 2 004 747
JP-A-56 018 904
JP-A-56 099 401
US-A- 3 870 795
US-A- 4 241 214

(56) References cited :
J. Soc. Cosmet. Chem. 31, 253-267 (September/Oktober 1980) "Marginalien zum Thema Konservierung I "
J. Soc. Cosmet. Chem. 31, 299-309 (1980) " Marginalien zum Thema Konservierung II "
"Seifen, Oele, Fette, Wachse", Nr. 8 (1985) entsprechend Vortrag des Autors auf Forum " Cosmeticum 1984" in Basel

(73) Proprietor : ROHM AND HAAS COMPANY
Independence Mall West
Philadelphia Pennsylvania 19105 (US)

(72) Inventor : Amick, David Richard
110 Devon Road
Chalfont, Pa. 18914 (US)

(74) Representative : Angell, David Whilton et al
ROHM AND HAAS (UK) LTD. European Operations Patent Department Lennig House
2 Mason's Avenue
Croydon CR9 3NB (GB)

EP 0 194 146 B2

## Description

This invention is concerned with stabilized non-aqueous solutions of 5-chloro-3-isothiazolones, their preparation, and their use in controlling living organisms.

Isothiazolones useful in the present invention are represented by the following structural formula:

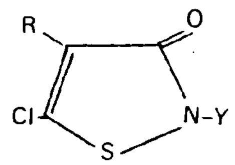

I

wherein

Y is an unsubstituted or substituted ($C_1$ to $C_8$)-alkyl, an unsubstituted or halo substituted ($C_2$ to $C_8$)-alkenyl or ($C_2$ to $C_8$)-alkynyl preferably ($C_2$ to $C_4$)-alkenyl or ($C_2$ to $C_4$)-alkynyl, unsubstituted ($C_5$ to $C_8$)-cycloalkyl, unsubstituted or substituted aralkyl or unsubstituted or substituted aryl;

R is hydrogen, halo, or a ($C_1$ to $C_4$)-alkyl.

Representative Y substituents include methyl, ethyl, propyl, isopropyl, butyl, hexyl, octyl, cyclohexyl, benzyl, 3,4-dichlorobenzyl, 4-methoxybenzyl, 4-chlorobenzyl, 3,4-dichlorophenyl, 4-methoxyphenyl, hydroxymethyl, chloromethyl and chloropropyl.

In many instances it is desirable to minimize or eliminate water, salt, and nitrate levels in isothiazolone biocides. For example, certain emulsions or dispersions require biocidal protection and are sensitive to shock resulting in a precipitate when salts, especially those containing divalent ions, are added. This precipitation precludes the use of biocides containing appreciable salt levels, especially in situations where mechanical stirring is not feasible.

In oils and fuels it is desirable to minimize water and salt content. Water in contact with the organic matter in fuel creates conditions suitable for biological growth and the formation of sludge. Also, salts in oils and fuel result in ignition deposits which lead to clogging and corrosion of various mechanical components.

In some cosmetic formulations, it is important to control water and salt content. Eliminating the presence of nitrate salts avoids the possibility of nitrosamine formation. Nitrosamines are suspected carcinogens.

This invention is directed to stable biocidal isothiazolone compositions in which (1) water is eliminated (2) neutralizing salt content can be eliminated and (3) nitrate stabilizer salts are eliminated.

This invention provides a method of making a stable non-aqueous solution of one or more isothiazolones of the formula:

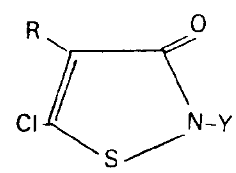

wherein

Y is an alkyl group of 1 to 8 carbon atoms optionally substituted with one or more hydroxy. halo, cyano, alkylamino, dialkylamino, arylamino, carboxy, carbalkoxy, alkoxy, aryloxy, alkylthio arylthio, isothiazolonyl, haloalkoxy, carbamoxy, morpholino, piperidino or pyrrolidino group, an unsubstituted or halo substituted alkenyl or alkynyl group of 2 to 8 carbon atoms, an unsubstituted cycloalkyl of 5 to 8 carbon atoms, an aralkyl group optionally substituted with one or more halo, nitro, ($C_1$ to $C_4$)-alkyl or ($C_1$ to $C_4$)-alkoxy groups, or an aryl group optionally substituted with one or more halo, cyano, nitro, ($C_1$ to $C_4$)alkyl, ($C_1$ to $C_4$)alkoxy, ($C_1$ to $C_4$)alkyl-acylamino, ($C_1$ to $C_4$)carbalkoxy or sulfamyl groups;

R is hydrogen, halo, or a ($C_1$ to $C_4$)alkyl; which comprises dissolving the isothiazolone in one or more hydroxy solvents of the formulae:

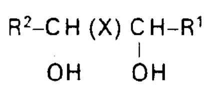 or 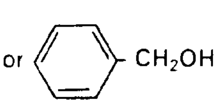 or $HO(C_3H_6O)_yH$

wherein $R^2$ and $R^1$ are hydrogen or lower alkyl, X is $-CH_2OCH_2-$ or $-(CH_2)_n-$ wherein n is an integer of 1 to 4 and y is an integer of 1 to 10, to form a solution containing 0.5 to 10 parts by weight isothiazolone and up to 99.5 parts by weight hydroxy solvent.

The preferred non-aqueous compositions contain from 3 to 10% by weight of one or more isothiazolones, and a stabilizing amount of hydroxy solvent(s) which may be present in an amount up to about 97% of a hydroxy solvent or mixture of said solvents.

In the stabilizing hydroxy solvents $R^2$ and $R^1$ are preferably hydrogen or methyl, ethyl, propyl, butyl or pentyl.

Preferred stabilizing solvents include propylene glycol, benzyl alcohol, dipropylene glycol, polypropylene glycol and 1,5-pentanediol.

The stabilizing solvent is employed in an amount of from about 89 to about 99.5% by weight of the three stated components, with the most preferred amount depending on the amount of isothiazolone desired.

Prior to this discovery it was known to employ organic solvents with metal nitrates (See especially U.S. Patent 3 870 795, Col. 3, lines 39-54). However, the solvents were not known or expected to be useful as stabilizers when used in the amounts to which this invention is limited. U.S. Patent 3 870 795, Col. 4, lines 35-45 discloses that non-aqueous solutions containing about 15% of 5-chloro-2-methyl-3-isothiazolone/2-methyl-3-isothiazolone (93:7) in dipropylene glycol completely decomposed in 28 days at 50 °C.

Japanese Patent Publications 21 564/85 and 12 243/83, similarly, disclose compositions containing isothiazolone salts and

2-hydroxymethyl-2-nitro-1,3-propanediol.

U.S. Patents 4 241 214 and 4 396 413 describe metal salt complexes of the isothiazolones of formula I and their use as effective biocidal agents. Current commercial products containing such isothiazolones are sold as aqueous solutions containing divalent nitrate salts which serve as stabilizers for the isothiazolones which would otherwise decompose upon storage.

By contrast, this invention enables the stabilization of isothiazolones without employing stabilization salts.

Hitherto in the preparation of the 5-chloro-3-isothiazolones, it was desired to partially neutralize the intermediate isothiazolone hydrochloride salt to obtain a more stable product; this resulted in the formation of a « neutralization salt » as a by-product. The present invention enables the elimination of the neutralization salt, by removing the hydrogen chloride formed in the preparation of the isothiazolone by treating the isothiazolone hydrochloride salt with organic base. Tertiary, organic bases are preferred, for example, trialkylamines such as trimethylamine, triethylamine and tripropylamine also cyclic tertiary amines such as pyridine. While an inorganic compound can be used to neutralize the hydrogen chloride, it dissolves and reacts very slowly in the « all organic » formulation, and results in the formation of a neutralization salt which is undesirable.

Typical ranges for the content of the components in the compositions are illustrated in the following Table I (all percentages are parts by weight of the three stated components).

Table I

| Isothiazolone | Hydroxy Solvent |
|---|---|
| 0.5–10 | up to 99.5 |
| Preferred<br>3.0–10.0 | up to 97.0 |

Solutions of isothiazolones are useful as water-cooling system microbicides, as preservatives for aqueous dispersions or organic polymers, as wood pulp white water slimicides, as cosmetic preservatives, as cutting oil, jet fuel and heating oil preservatives. Solution of isothiazolones are also applied to a solid substrate, such as fabric, leather or wood, as a preservative.

The products of this invention are especially useful as preservatives for the following: 1. Cosmetics, as the presence of nitrates (which under certain conditions in the presence of amines or amine precursors may lead to the formation of nitrosamines) can be eliminated. 2. Oils and fuels. since added salts and moisture can be eliminated thus preventing potential corrosion, deposition or sludge formation. 3. Emulsions and dispersions that are sensitive to the addition of salts. Examples of these emulsions and dispersions are those contained in a wide variety of products, such as paints, cosmetics, floor polishes and binders.

The following Example will further illustrate this invention. All parts and percentages are by weight and all temperatures in degrees Centigrade, unless otherwise stated.

In interpreting the results in the Example it should be noted that our studies show that the temperature of

55 °C causes an acceleration effect so that one week at that temperature is equivalent to about 3 months at 25 °C; two weeks is equivalent to about 6 months; three weeks is equivalent to 9 months; and four weeks is equivalent to about 12 months, and so on. A temperature of 65 °C causes an acceleration effect so that one week at that temperature is equivalent to about 7 months at 25 °C; two weeks is equivalent to 14 months; three weeks is equivalent to 21 months; four weeks is equivalent to 28 months, and so on We consider that any product which does not show signs of decomposition in one year (4 weeks at 55 °C or about 2 weeks at 65 °C) should be regarded as a stable product.

Example 1

- Stability (by HPLC) of 5-chloro-2-methylisothiazolin-3-one in Organic Solvents at 65 °C (« No $H_2O$ ») (1 % AI to start)

| Solvent | 2 days | 2 weeks | 4 weeks | 6 weeks |
|---|---|---|---|---|
| Dipropylene glycol | P | P | P | P |
| Polypropylene glycol (MW=2000) | P | P | P | P |
| Propylene glycol | P | P | F | F |
| Ethylene glycol | P | P | F | F |
| Diethylene glycol | P | P | F | F |
| Triethylene glycol | P | P | F | F |
| 1,5-Pentanediol | P | P | P | F |
| 2,3-Pentanediol | P | F | F | F |

AI (Active Ingredient) determined by HPLC (HPLC=High Pressure Liquid Chromatography)
P = essentially no loss of AI
F = AI totally decomposed

**Claims**

1. A method of making a stable non-aqueous solution of one or more isothiazolones of the formula:

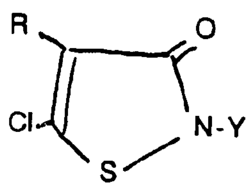

wherein,
Y is an alkyl group of 1 to 8 carbon atoms optionally substituted with one or more hydroxy, halo, cyano, alkylamino, dialkylamino, arylamino, carboxy, carbalkoxy, alkoxy, aryloxy, alkylthio, arylthio, isothiazolonyl, haloalkoxy, carbamoxy, morpholino, piperidino or pyrrolidino group, an unsubstituted or halo substituted alkenyl or alkynyl group of 2 to 8 carbon atoms, an unsubstituted cycloalkyl of 5 to 8 carbon atoms, an aralkyl group optionally substituted with one or more halo, nitro, ($C_1$ to $C_4$)alkyl or ($C_1$ to $C_4$)alkoxy groups, or an aryl group optionally substituted with one or more halo, cyano, nitro, ($C_1$ to $C_4$)alkyl, ($C_1$ to $C_4$)carbalkoxy or sulfamyl groups; R is hydrogen, halo, or a ($C_1$ to $C_4$)alkyl group; which comprises dissolving the isothiazolone in one or more hydroxy solvents of the formulae:

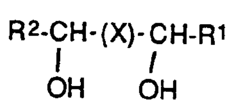 or 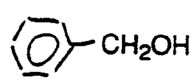 or $HO(C_3H_6O)_yH$

wherein $R^2$ and $R^1$ are hydrogen or ($C_1$ to $C_6$)alkyl, X is $-CH_2OCH_2-$ or $-(CH_2)_n-$ wherein n is an integer of

1 to 4 and y is an integer of 1 to 150, to form a solution containing 0.5 to 10 parts by weight isothiazolone, up to 99.5 parts by weight hydroxy solvent and no stabilisation salt.

2. The use of one or more hydroxy solvents of the formulae defined in Claim 1 replacement for nitrate stabilisation salt in stable non-aqueous isothiazolone solutions as prepared according to Claim 1.

3. A method or use according to any preceding Claim wherein the isothiazolone content is from 3 to 10% by weight and the solvent content is from 90 to 97% by weight.

4. A method or use according to any preceding Claim wherein the solvent comprises propylene glycol, polypropylene glycol, 1,5-pentanediol, benzyl alcohol or, preferably dipropylene glycol.

5. A method or use according to any preceding Claim wherein the isothiazolone comprises 5-chloro-2-methyl isothiazolin-3-one.

6. The use of a solution prepared by a process according to any of Claims 1 or 3 to 5, as a biocide or preservative in a cosmetic, oil, fuel, paint, floor polish or binder formulation.

## Patentansprüche

1. Verfahren zur Herstellung einer stabilen, nicht wäßrigen Lösung aus 1 oder mehreren Isothiazolon(en) der Formel

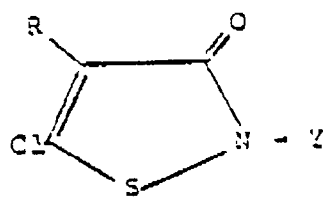

worin

Y ein Alkyl mit 1 bis 8 Kohlenstoffatomen, gegebenenfalls substituiert mit 1 oder mehreren Hydroxy-, Halogen-, Cyano-, Alkylamino-, Dialkylamino-, Arylamino-, Carboxy-, Carbalkoxy-, Alkoxy-, Aryloxy-, Alkylthio-, Arylthio-, Isothiazolonyl-, Halogenalkoxy-, Carbamoxy-, Morpholino-, Piperidino- oder Pyrrolidinogruppe(n), eine nichtsubstituierte oder Halogen-substituierte Alkenyl- oder Alkinylgruppe mit 2 bis 8 Kohlenstoffatomen, ein nichtsubstituiertes Cycloalkyl mit 5 bis 8 Kohlenstoffatomen, eine Aralkylgruppe, gegebenenfalls substituiert mit 1 oder mehreren Halogen-, Nitro-, ($C_1$-$C_4$)-Alkyl- oder ($C_1$-$C_4$)-Alkoxygruppe(n), oder eine Arylgruppe, gegebenenfalls substitulert mit 1 oder mehreren Halogen-, Cyano-, Nitro-, ($C_1$-$C_4$)-Alkyl-, ($C_1$-$C_4$)-Carbalkoxy- oder Sulfamylgruppe(n), ist,

R Wasserstoff, Halogen oder eine ($C_1$-$C_4$)-Alkylgruppe ist durch Auflösen des Isothiazolones in 1 oder mehreren Hydroxylösemittel(n) der Formel

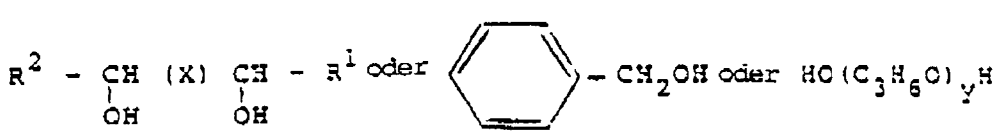

worin $R^2$ und $R^1$ Wasserstoff oder ($C_1$-$C_6$)-Alkyl sind, X $-CH_2OCH_2-$ oder $-(CH_2)_n-$ ist, wobei n eine ganze Zahl von 1 bis 4 ist und y eine ganze Zahl von 1 bis 150 ist, um eine Lösung auszubilden, die 0,5 bis 10 Gewichtsteile Isothiazolon und bis 99,5 Gewichtsteile Hydroxylösemittel enthält.

2. Verwendung von 1 oder mehreren Lösemittel(n) der in Anspruch 1 angegebenen Formeln als vollständigen Ersatz von stabilisierendem Mitratsalz in stabilen, nicht wäßrigen Isothiazolonlösungen, hergestellt nach Anspruch 1.

3. Verfahren oder Verwendung nach einem der vorstehenden Ansprüche, dadurch gekennzeichnet, daß der Isothiazolongehalt von 3 bis 10 Gewichtsprozent und der Lösemittelgehalt von 90 bis 97 Gewichtsprozent beträgt.

4. Verfahren oder Verwendung nach einem der vorstehenden Ansprüche, dadurch gekennzeichnet, daß das Lösemittel Propylenglykol, Polypropylenglykol, 1,5-Pentandiol, Benzylalkohol oder vorzugsweise Dipropylenglykol enthält.

5. Verfahren oder Verwendung nach einem der vorstehenden Ansprüche, dadurch gekennzeichnet, daß das Isothiazolon 5-Chlor-2-methyl-isothiazolin-3-on enthält.

6. Verwendung nach einem Verfahren gemäß einem der Ansprüche 1 oder 3 bis 5 hergestellten Lösung als Biozid oder Schutzmittel in einer Kosmetik-, Öl-, Treibstoff-, Anstrich-, Fußbodenpolier- oder Bindemittelformulierung.

**Revendications**

1. Procédé pour préparer une solution non aqueuse stable d'une ou plusieurs isothiazolones de formule :

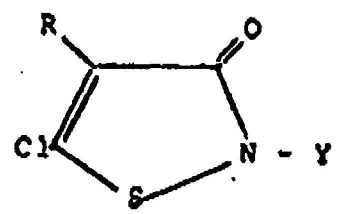

dans laquelle

Y est un groupe alcoyle de 1 à 8 atomes de carbone facultativement substitué par un ou plusieurs groupes hydroxy, halogéno, cyano, alcoylamino, diacoylamino arylamino, carboxy, carbalcoxy, alcoxy, aryloxy, alcoylthio, arylthio, isothiazolonyles, halogénoalcoxy, carbamoxy, morpholino, pipéridino ou pyrrolidino, un groupe alcényle ou alcynyle de 2 à 8 atomes de carbone non substitué ou halogéno-substitué, un groupe cycloalcoyle non substitué de 5 à 8 atomes de carbone, un groupe aralcoyle facultativement substitué par un ou plusieurs groupes halogéno, nitro, alcoyles ($C_1$ à $C_4$) ou alcoxy ($C_1$ à $C_4$), ou un groupe aryle facultativement substitué par un ou plusieurs groupes halogéno, cyano, nitro, alcoyles ($C_1$ à $C_4$), carbalcoxy ($C_1$ à $C_4$) ou sulfamyles ;

R est un hydrogène, un halogéno, ou un groupe alcoyle ($C_1$ à $C_4$) ;

qui comprend la dissolution de l'isothiazolone dans un ou plusieurs solvants hydroxylés des formules :

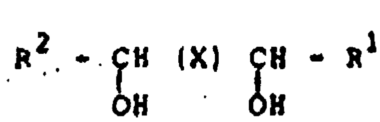
ou
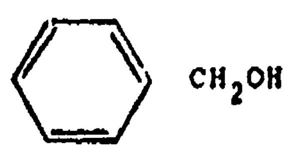

dans lesquelles $R^2$ et $R^1$ sont un hydrogène ou un alcoyle ($C_1$ à $C_6$), X est $-CH_2OCH_2-$ ou $-(CH_2)_n-$ où n est un entier de 1 à 4 et y est un entier de 1 à 150, pour former une solution contenant 0,5 à 10 parties en poids d'isothiazolone et jusqu'à 99,5 parties en poids de solvant hydroxy et aucun sel de stabilisation.

2. Utilisation d'un ou de plusieurs solvants hydroxy des formules définies dans la revendication 1 en tant que remplacement total pour le sel de stabilisation de nitrate dans des solutions d'isothiazolone non aqueuse stable telle que préparée selon la revendication 1.

3. Procédé ou utilisation selon l'une quelconque des revendications précédentes dans laquelle la teneur en isothiazolone est de 3 à 10% en poids et la teneur en solvant est de 90 à 97% en poids.

4. Procédé ou utilisation selon l'une quelconque des revendications précédentes dans laquelle le solvant comprend du propylèneglycol, du polypropylèneglycol, de 1,5-pentanediol, l'alcool benzylique ou de préférence le dipropylèneglycol.

5. Procédé ou utilisation selon l'une quelconque des revendications précédentes dans laquelle l'isothiazolone comprend la 5-chloro-2-méthyle isothiazoline 3-one.

6. Utilisation d'une solution préparée par un procédé selon l'une quelconque des revendications 1 ou 3 à 5, en tant que biocide ou agent de conservation dans une formulation de cosmétique, d'huile, de combustible, de peinture, de vernis pour sol ou de liant.